# EUROPÄISCHE PATENTSCHRIFT

(11) **EP 0 528 268 B1**
(45) Veröffentlichungstag und Bekanntmachung des Hinweises auf die Patenterteilung: **08.01.1997**
(21) Anmeldenummer: 92113314.6
(22) Anmeldetag: 05.08.1992
(51) Int. Cl.: C07C 69/712, C09K 19/06, C09K 19/12, C07C 69/94, C07C 69/92, C09K 19/58, C09K 19/20, C09K 19/04, C08G 63/02, C08F 20/30

(54) **Optisch aktive Phenoxi-propionsäureester**
Optically active esters of phenoxypropionic acid
Esters d'acide phenoxy propionique optiquement actifs

(30) Priorität: 16.08.1991 DE 4126996
(43) Veröffentlichungstag der Anmeldung: 24.02.1993
(73) Patentinhaber: BASF Aktiengesellschaft, 67063 Ludwigshafen (DE)
(72) Erfinder: Bach, Volker, Dr., W-6730 Neustadt (DE); Brox, Wolfgang, Dr., W-6900 Heidelberg (DE); Etzbach, Karl-Heinz, Dr., W-6710 Frankenthal (DE); Paul, Axel, Dr., W-6840 Lampertheim (DE); Siemensmeyer, Karl, Dr., W-6710 Frankenthal (DE)

(56) Entgegenhaltungen:
- EP-A- 0 196 070
- EP-A- 0 219 481
- EP-A- 0 331 367
- JOURNAL OF THE AMERICAN CHEMICAL SOCIETY Bd. 108, Nr. 17 , 20. August 1986 , WASHINGTON, DC US Seiten 5210 - 5221 D.M.WALBA 'Design and synthesis of a new ferroelectric liquid crystal family. Liquid crystals containing a nonracemic 2-alkoxy-1-propoxy unit'

## Beschreibung

Die Erfindung betrifft optisch aktive Verbindungen der Formel I 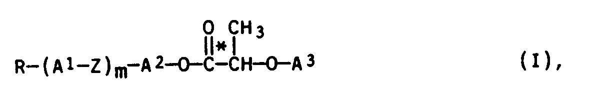 worin die Indices und die Variablen die folgende Bedeutung haben:
- R: eine C₁-C₁₂-Alkyl- oder -Perfluoralkyl-Gruppe, in der auch eine oder zwei nicht benachbarte CH₂- bzw. CF₂-Gruppen durch O-Atome und/oder -CO-Gruppen und/oder -CO-O-Gruppen und/oder -CH=CH-Gruppen und/oder -CH-Halogen- und/oder -CHCN-Gruppen und/oder -O-CO-CH-Halogen- und/oder -O-CO-CHCN-Gruppen ersetzt sein können, oder eine C₁-C₁₂-Alkylgruppe, die endständig eine chemisch reaktive Gruppe tragen kann und in der eine CH₂-Gruppe durch ein O-Atom ersetzt sein kann,
- A¹ und A²: unabhängig voneinander unsubstituiertes oder durch ein oder zwei F- und/oder Cl- und/oder Br-Atome und/oder CH₃-Gruppen und/oder CN-Gruppen substituiertes 1,4-Phenylen, worin auch eine oder zwei CH-Gruppen durch N ersetzt sein können, 1,4-Cyclohexylen, worin auch eine oder zwei nicht benachbarte CH₂-Gruppen durch O-Atome und/oder S-Atome ersetzt sein können, Piperidin-1,4-diyl, 1,4-Bicyclo(2,2,2)octylen, Naphthalin-2,6-diyl, Decahydronaphthalin-2,6-diyl oder 1,2,3,4-Tetrahydronaphthalin-2,6-diyl,
- A³: gegebenenfalls substituiertes Phenyl,
- Z: -CO-O-, -O-CO-, -CH₂CH₂-, -OCH₂-, -CH₂O- -C≡C- oder eine Einfachbindung und
- m: 0, 1, 2 oder 3,
mit der Maßgabe, daß für den Fall, daß m = 2 oder 3 ist, die Reste A¹ und Z in den einzelnen Gruppen -(A¹-Z) unabhängig voneinander gleich oder verschieden sein können.

Weiterhin betrifft die Erfindung Polymere II, die dadurch gekennzeichnet sind, daß sie aus Verbindungen der Formel I hergestellt werden oder diese als Bestandteil enthalten.

Außerdem betrifft die Erfindung niedermolekulare, glasartig erstarrende Verbindungen XI, die dadurch gekennzeichnet sind, daß sie mehrere Gruppen, die sich von den Verbindungen der Formel I ableiten, als Substituenten tragen.

Flüssigkristalle haben in jüngerer Zeit Eingang in verschiedenste technische Gebiete gefunden, in denen elektrooptische und Anzeigevorrichtungs-Eigenschaften gefragt sind (z.B. in Uhren-, Taschenrechner- und Schreibmaschinenanzeigen). Diese Anzeigevorrichtungen beruhen auf den dielektrischen Ausrichtungseffekten in den nematischen, cholesterischen und/oder smektischen Phasen der flüssigkristallinen Verbindungen, wobei - verursacht durch die dielektrische Anisotropie - die molekulare Längsachse der Verbindungen eine bestimmte Ausrichtung in einem angelegten elektrischen Feld einnimmt. Die üblichen Schaltzeiten bei diesen Anzeigevorrichtungen sind für viele andere potentielle Anwendungsgebiete von Flüssigkristallen, die an sich für die Technik wegen ihrer einzigartigen Eigenschaften sehr vielversprechende chemische Verbindungen sind, eher zu lang. Dieser Nachteil macht sich insbesondere dann bemerkbar, wenn - was bei größeren Anzeigeelementflächen zwangsläufig der Fall ist - eine große Anzahl von Bildpunkten angesteuert werden muß, wodurch die Bildwiederholfrequenz und somit die Bildqualität von Geräten mit größeren Flächen wie Videogeräten, Oszillographen oder Fernseh-, Radar-, EDV- oder Schreibautomaten-Bildschirmen zu niedrig wären.

Neben den nematischen und cholesterischen Flüssigkristallen haben seit einigen Jahren in zunehmendem Maß für Praxisanwendungen auch ferroelektrische smektische Flüssigkristall-Phasen an Bedeutung gewonnen.

Chirale getiltete smektische flüssigkristalline Phasen mit ferroelektrischen Eigenschaften können hergestellt werden, indem man Basis-Mischungen mit einer oder mehreren getilteten smektischen Phasen mit einem geeigneten chiralen Dotierstoff versetzt (L.A. Beresnev et al., Mol. Cryst. Liq. Cryst. 89, 327 (1982); H.R. Brand et al., J. Physique 44, (lett.) L-771 (1983)). Solche Phasen können als Dielektrika für schnell schaltende Displays verwendet werden, die auf dem von Clark und Lagerwall beschriebenen Prinzip der SSFLC-Technologie (N.A. Clark und S.T. Lagerwall, Appl. Phys. Lett. 36, 899 (1980); US-A-4 367 924) auf der Basis der ferroelektrischen Eigenschaften der chiral getilteten Phase beruhen. In dieser Phase sind die langgestreckten Moleküle in Schichten angeordnet, wobei die Moleküle einen Tiltwinkel zur Schichtennormalen aufweisen. Beim Fortschreiten von Schicht zu Schicht ändert sich die Tiltrichtung um einen kleinen Winkel bezüglich einer senkrecht zu den Schichten stehenden Achse, so daß eine Helixstruktur ausgebildet wird. In Displays, die auf dem Prinzip der SSFLC-Technologie beruhen, sind die smektischen Schichten senkrecht zu den Platten der Zelle angeordnet. Die helixartige Anordnung der Tiltrichtung der Moleküle wird durch einen sehr geringen Abstand der Platten (ca. 1 bis 2 µm) unterdrückt. Dadurch werden die Längsachsen der Moleküle gezwungen, sich in einer Ebene parallel zu den Platten der Zelle anzuordnen, wodurch zwei elektrisch und optisch unterscheidbare Tiltorientierungen entstehen. Durch Anlegen eines geeigneten elektrischen Wechselfeldes kann in der eine spontane Polarisation aufweisenden flüssigkristallinen Phase zwischen diesen beiden Zuständen hin- und hergeschaltet werden. Dieser Schaltvorgang ist wesentlich schneller als bei herkömmlichen verdrillten Zellen (TN-LCD's), die auf nematischen Flüssigkristallen basieren.

Clark und Lagerwall konnten zeigen, daß die Verwendung solcher Flüssigkristallsysteme in sehr dünnen Zellen zu optoelektrischen Schalt- oder Anzeigeelementen führt, die im Vergleich zu den herkömmlichen TN ("twisted nematic")-Zellen um bis zu etwa einem Faktor 1000 schnellere Schaltzeiten haben (vgl. z.B. Lagerwall et al. "Ferroelectric Liquid Crystals for Displays", SID Symposium, October Meeting 1985, San Diego, Ca., USA). Aufgrund dieser und anderer günstiger Eigenschaften, z.B. der bistabilen Schaltmöglichkeiten und des nahezu blickwinkelunabhängigen Kontrastes, sind ferroelektrische Flüssigkristalle grundsätzlich für die obengenannten Anwendungsgebiete, z.B. über eine Matrixansteuerung, gut geeignet.

In der DE-A-3 930 667 wird ein Verfahren zur reversiblen oder irreversiblen Erzeugung einer Abbildung durch bildmäßige Einwirkung von Energie auf eine Aufzeichnungsschicht in Anwesenheit oder Abwesenheit eines elektrischen und/oder magnetischen Feldes beschrieben, wodurch auf der Oberfläche der Aufzeichnungsschicht ein der bildmäßigen Einwirkung der Energie entsprechendes Muster aus Oberflächenladungen resultiert. Hierbei werden chirale, flüssigkristalline Polymere eingesetzt.

Für die praktische Verwendung von ferroelektrischen Flüssigkristallen in optoelektrischen Anzeigen sind aber die Schaltzeiten der bisher bekannten Verbindungen noch zu lang. Es besteht daher ein Bedarf an neuen Verbindungen mit wesentlich kürzeren Schaltzeiten.

Der Erfindung lag daher die Aufgabe zugrunde, neue stabile flüssigkristalline Verbindungen zu finden, die als Komponenten flüssigkristalliner Phasen geeignet sind oder zum Aufbau von flüssigkristallinen Polymeren dienen können, die flüssigkristalline Phasen mit Schichtstruktur bilden und sehr kurze Schaltzeiten aufweisen.

Diese Aufgabe konnte durch die erfindungsgemäßen Verbindungen I, II und XI gelöst werden.

Der Einfachheit halber bedeuten im folgenden Ph eine 1,4-Phenylengruppe, worin auch eine oder zwei CH-Gruppen durch N ersetzt sein können.

Vor- und nachstehend haben R, m, A¹, A², A³, Ph und Z die oben angegebene Bedeutung, sofern nicht ausdrücklich etwas anderes vermerkt ist. In den nachstehenden Formeln bezeichnen Z¹ und Z² jeweils eine Gruppe Z, wobei Z¹ und Z² gleich oder voneinander verschieden sein können.

m bedeutet vorzugsweise 1 oder 2.

Die Verbindungen der Formel I umfassen dementsprechend insbesondere Verbindungen der Formeln Ia und Ib (m = 1 bzw. m = 2): 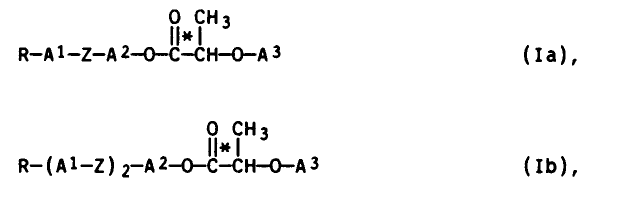 A¹ und A² stehen bevorzugt für Ph, wobei Ph vorzugsweise eine 1,4-Phenylengruppe bedeutet.

Unter diesen Verbindungen sind diejenigen der Formeln Ib besonders bevorzugt. Die Verbindungen der Formel Ib umfassen beispielsweise solche der Teilformeln Ib1 bis Ib6:

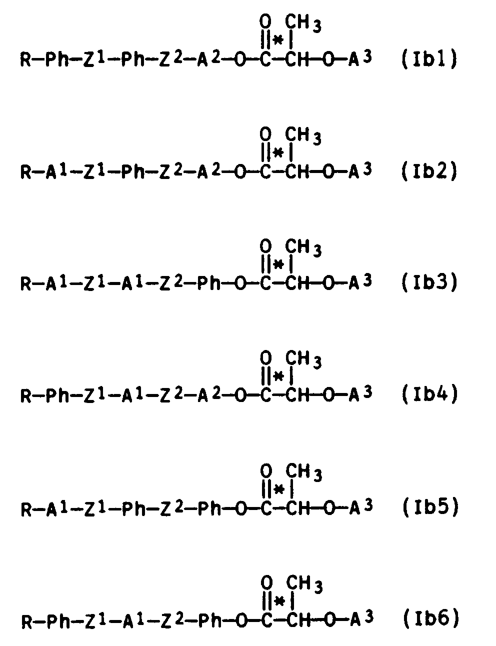

In den Verbindungen der Formel (Ib3) können die beiden Gruppen A¹ gleich oder voneinander verschieden sein. Besonders bevorzugt sind Verbindungen der Formel I, in denen Z bzw. Z¹ und Z² -CO-O-, -O-CO- oder eine Einfachbindung bedeuten.

A³ steht bevorzugt für eine unsubstituierte oder einen oder zwei Substituenten S¹ und S² tragende Phenylgruppe, wobei S¹ und S² vorzugsweise und unabhängig voneinander C₁-C₁₀-Alkyl, C₁-C₁₀-Alkoxy, Fluor, Chlor oder Brom bedeuten.

In den vor- und nachstehenden Formeln für die Verbindungen gemäß Formel I sind die Alkylreste R, in denen auch eine CH₂-Gruppe durch ein O-Atom ersetzt sein kann, vorzugsweise geradkettig. Bevorzugt haben sie 5 bis 11 C-Atome und bedeuten demnach Pentyl, Hexyl, Heptyl, Octyl, Nonyl, Decyl, Undecyl, Pentoxy, Hexoxy, Heptoxy, Octoxy, Nonoxy, Decoxy oder Undecoxy.

Die vorangehend beschriebenen Alkylreste R sind endständig zur Verknüpfungsstelle vorzugsweise durch reaktive Gruppen R¹ substituiert, wie etwa
CH₂=CH-, HO-, Cl-, Br-, 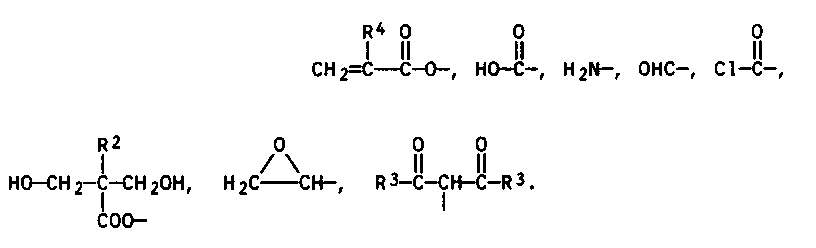
- R²: bedeutet in den oben angegebenen Formeln H, CH₃ oder C₂H₅,
- R³: bedeutet in den oben angegebenen Formeln -OH oder -Cl,
- R⁴: bedeutet in den oben angegebenen Formeln H, Cl, CH₃,
Reaktive endständige Gruppen R¹ in den Alkylresten R sind bevorzugt Acrylat, Methacrylat, Chloracrylat, Hydroxy oder Ethenyl.

Unter den Polymeren II sind solche Verbindungen besonders bevorzugt, die durch Polymerisierung (beispielsweise radikalische Polymerisation, Polykondensation, polymeranaloge Reaktion) aus solchen Verbindungen der Formel I, in denen die Reste R mit endständigen chemisch reaktiven Gruppen R¹ substituiert sind, hergestellt werden.

Beispiele solcher bevorzugter Polymerer II sind in den Formeln IIa1 bis IId6 aufgeführt. Der Index n ist dabei eine natürliche Zahl, die die Kettenlänge der Polymerhauptkette kennzeichnet.

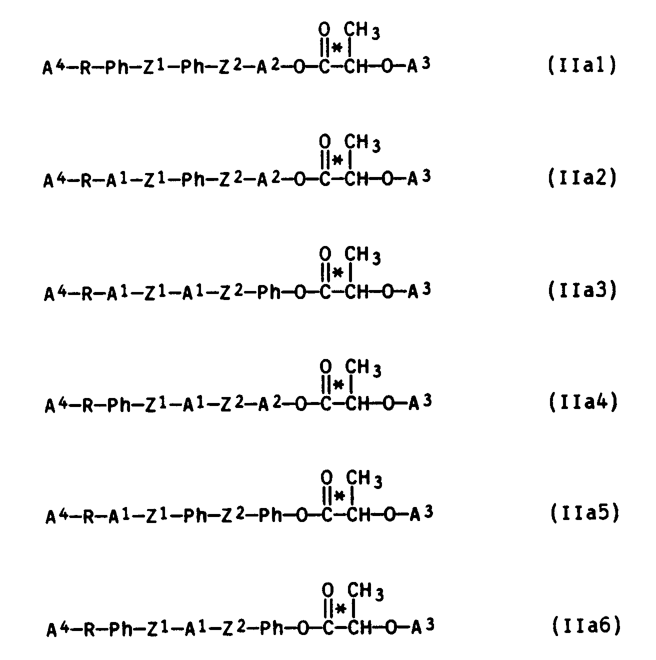

In den Formeln (IIa1) bis (IIa6) bedeutet A⁴ einen Rest der Formel (III)

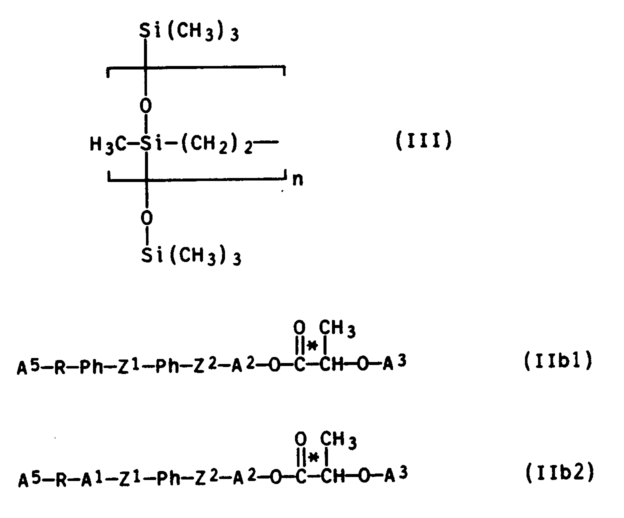

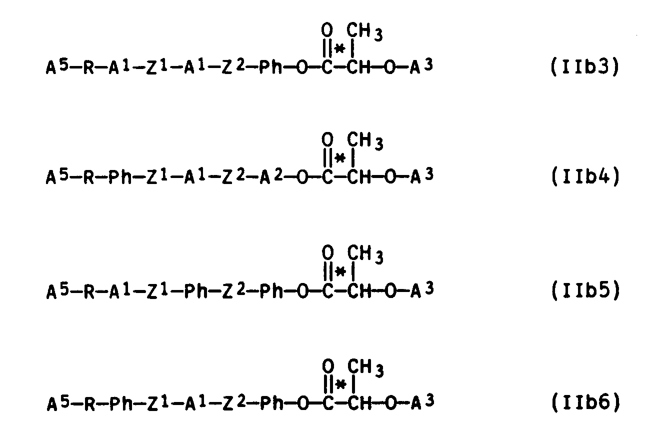

In den Formeln (IIb1) bis (IIb6) bedeutet A⁵ einen Rest der Formel IV 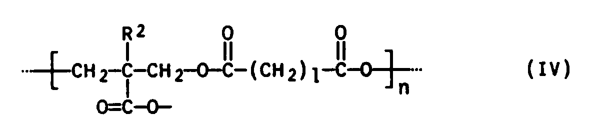 wobei l für eine natürliche Zahl von 1 bis 20 steht.

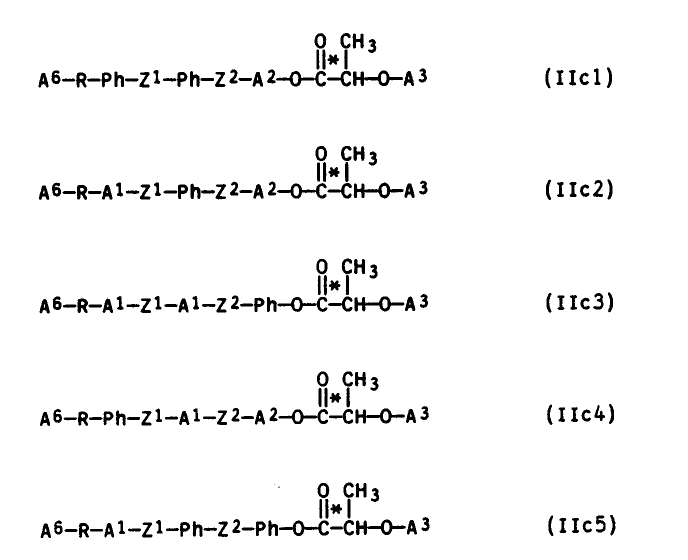

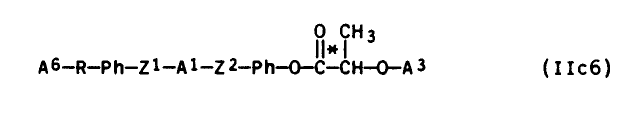

In den Formeln (IIc1) bis (IIc6) bedeutet A⁶ einen Rest der Formel V

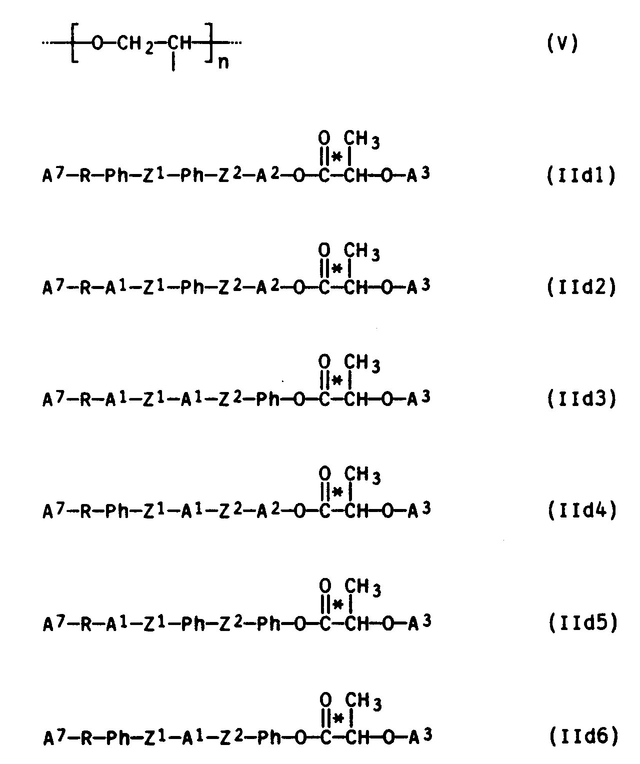

Ganz besonders bevorzugt sind Verbindungen der Formeln (IId1) bis (IId6), in denen A⁷ für einen Rest der Formel (VI) steht.

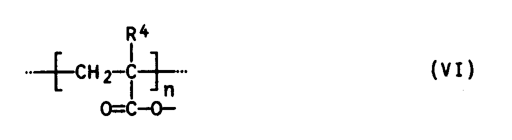

Die Herstellung der erfindungsgemäßen Verbindungen I und II erfolgt nach an sich bekannten Verfahren, wie sie beispielsweise in der DE-A-38 23 154, EP-A-258 898, EP-A-274 128, DE-A-38 27 601 und DE-A-39 17 196 angegeben sind.

Dabei wird beispielsweise bei der Herstellung der Verbindungen (Ib7) und (IId7) die chirale Phenoxypropionsäure VII direkt als Säure oder Säurechlorid eingesetzt und so das chirale Kohlenstoffatom eingebaut.

### Beispielhaftes Syntheseschema:

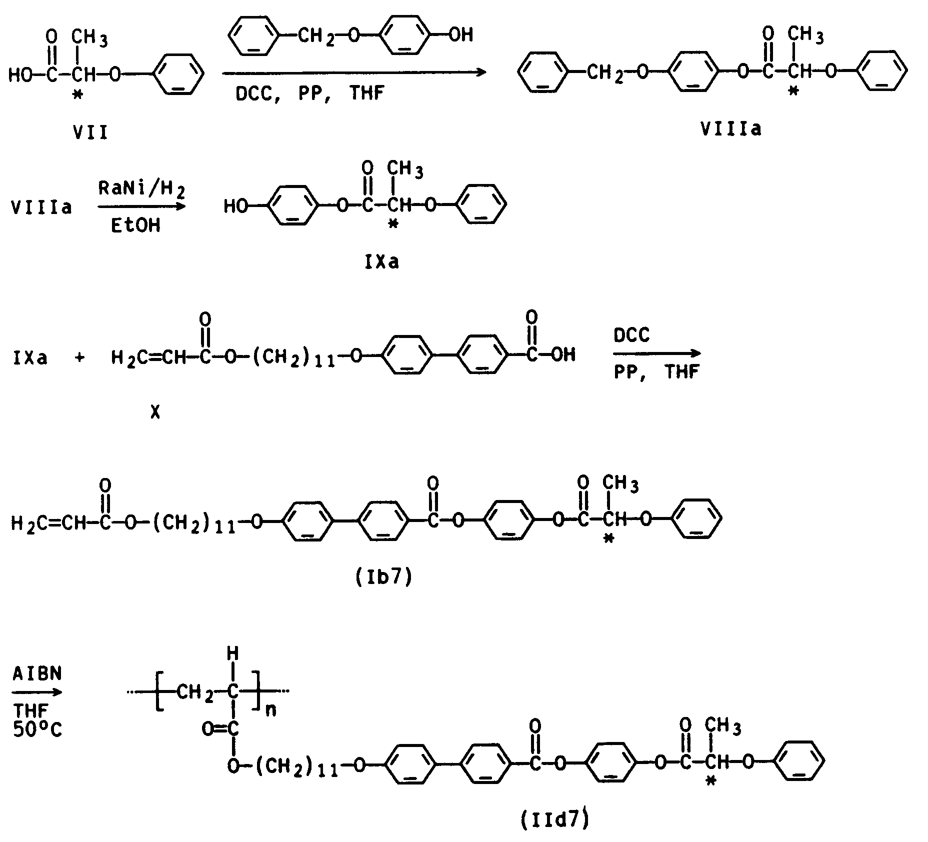

Darin haben die verwendeten Abkürzungen die folgende Bedeutung:
- AIBN: 2,2'-Azo-bis-(isobutyronitril)
- DCC: Dicyclohexylcarbodiimid
- EtOH: Ethanol
- PP: Pyrrolidino-pyridin
- RaNi: Raney-Nickel
- THF: Tetrahydrofuran

Die erfindungsgemäßen niedermolekularen, glasartig erstarrenden Verbindungen XI sind durch chemische Reaktion von mehrfach reaktiven Verbindungen Z³ mit solchen optisch aktiven Verbindungen I erhältlich, die reaktive Gruppen R¹ tragen. Sie können beispielsweise durch Veresterung der erfindungsgemäßen Verbindungen I mit mehrwertigen Säuren oder Alkoholen oder durch Addition der Verbindungen I an mehrwertige Isocyanate gewonnen werden.

Die zentralen Gruppen der Verbindung XI leiten sich beispielsweise von folgenden Verbindungen Z³ ab:
von aliphatischen Alkoholen wie
   Glycerin,
   1,2,4-Butantriol,
   2-Methyl-2-hydroxymethyl-1,3-propandiol,
   2-Ethyl-2-hydroxymethyl-1,3-propandiol,
   1,2,3,4-Butantetraol,
   Pentaerythrit,
   Xylit,
   Mannit und
   Sorbit,
von aliphatischen Carbonsäuren wie
   1,2,3-Propantricarbonsäure,
   1,1,4-Butantricarbonsäure,
   1,2,3,4-Butantetracarbonsäure,
   Zitronensäure und
   2-Hydroxy-nonadecyl-1,2,3-tricarbonsäure,
von cycloaliphatischen Alkoholen mit 5- oder 6-Ringgliedern wie
   1,2,3,4-Tetrahydroxy-cyclopentan,
   1,2,3-Trihydroxy-cyclohexan,
   1,2,4-Trihydroxy-cyclohexan,
   1,3,5-Trihydroxy-cyclohexan,
   1,2,3,4-Tetrahydroxy-cyclohexan,
   1,2,3,5-Tetrahydroxy-cyclohexan,
   1,2,4,5-Tetrahydroxy-cyclohexan,
   1,2,3,4,5-Pentahydroxy-cyclohexan und
   1,2,3,4,5,6-Hexahydroxy-cyclohexan,
von cycloaliphatischen Carbonsäuren mit 5- oder 6-Ringgliedern wie
   1,2,3-Cyclopentantricarbonsäure,
   1,2,4-Cyclopentantricarbonsäure,
   2-Methyl-1,2,3-cyclopentantricarbonsäure,
   3-Methyl-1,2,4-cyclopentantricarbonsäure,
   1,1,2,2-Cyclopentantetracarbonsäure,
   1,2,2,4-Cyclopentantetracarbonsäure,
   1,1,3,3-Cyclopentantetracarbonsäure,
   1,2,3,4-Cyclopentantetracarbonsäure,
   1,2,3,4,5-Cyclopentanpentacarbonsäure,
   1,1,4-Cyclohexantricarbonsäure,
   1,2,4-Cyclohexantricarbonsäure,
   1,3,5-Cyclohexantricarbonsäure,
   1,1,3,3-Cyclohexantetracarbonsäure,
   1,1,4,4-Cyclohexantetracarbonsäure,
   1,2,3,4-Cyclohexantetracarbonsäure,
   1,2,4,5-Cyclohexantetracarbonsäure,
   1,1,3,3,5-Cyclohexanpentacarbonsäure und
   1,2,3,4,5,6-Cyclohexanhexacarbonsäure,
von Alkoholaminen wie
   Triethanolamin,
   Triisopropanolamin und
   Aminoethylethanolamin,
von Triazinderivaten wie
   Cyanursäure,
   Thiocyanursäure,
   Melamin und
   Trishydroxyethylisocyanurat,
von Isocyanaten wie
   Tetramethylendiisocyanat,
   Hexamethylendiisocyanat (HDI),
   Dodecamethylendiisocyanat,
   1,4-Diisocyanatocyclohexan,
   1-Isocyanato-3,5,5-trimethyl-5-isocyanatomethylcyclohexan (Isophoron diisocyanat, IPDI),
   4,4'-Diisocyanatodicyclohexylmethan (HMDI),
   4,4'-Diisocyanatodicyclohexylpropan-(2,2),
   1,4-Diisocyanatobenzol,
   2,4-Diisocyanatotoluol,
   2,6-Diisocyanatotoluol,
   4,4'-Diisocyanatodiphenylmethan,
   p-Xylylendiisocyanat sowie
   die aus der Polyurethanchemie bekannten höherfunktionellen Polyisocyanate.

Beispiele bevorzugter Verbindungen XI sind in den Formeln (XIa1)-(XId6) aufgeführt:

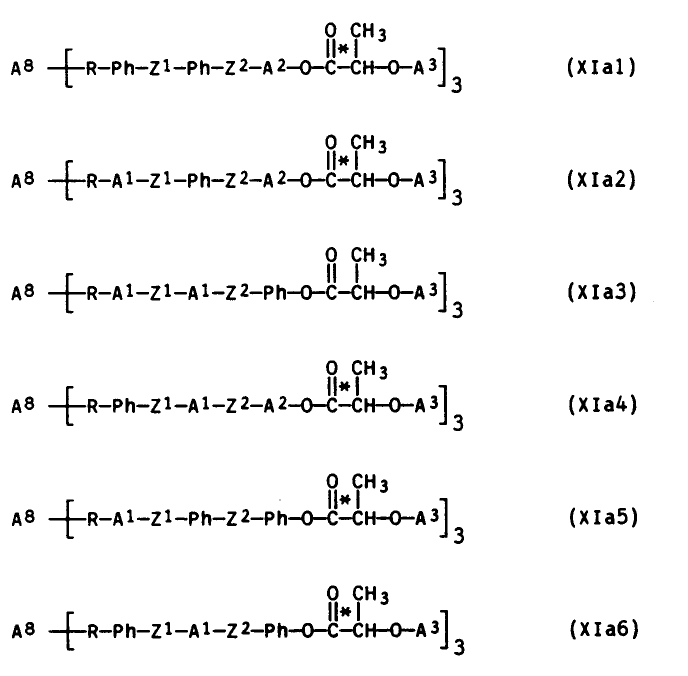

In den besonders bevorzugten Formeln (XIa1)-(XIa6) bedeutet A⁸ einen Rest der Formel XII:

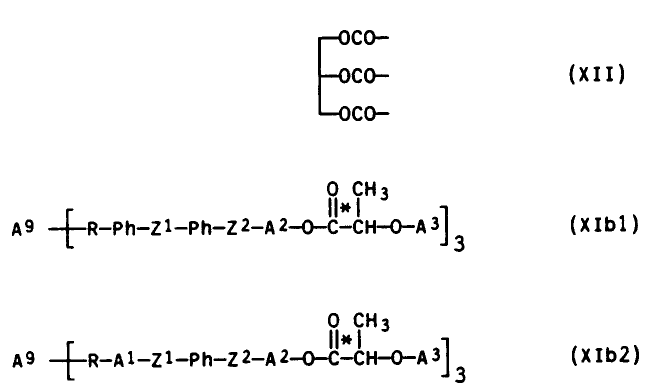

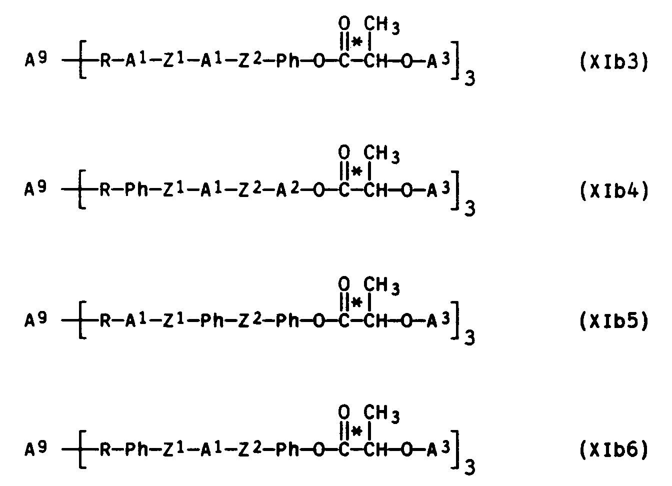

In den Formeln (XIb1)-(XIb6) bedeutet A⁹ einen Rest der Formel XIII:

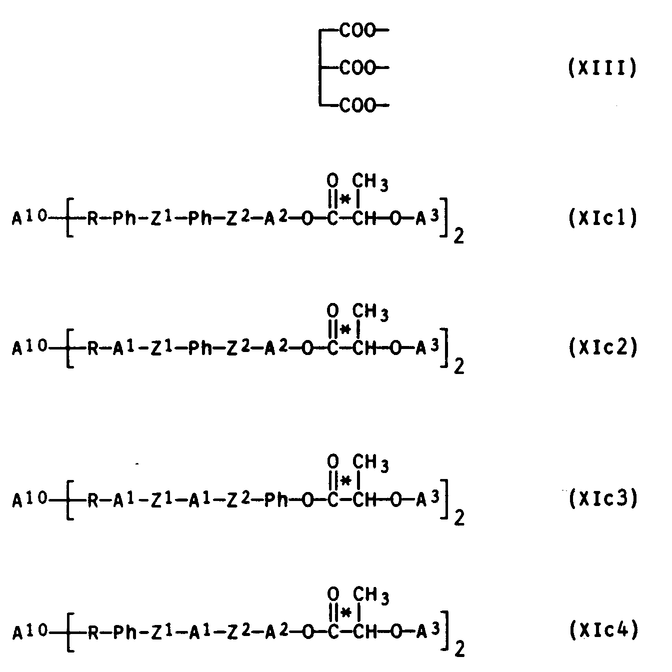

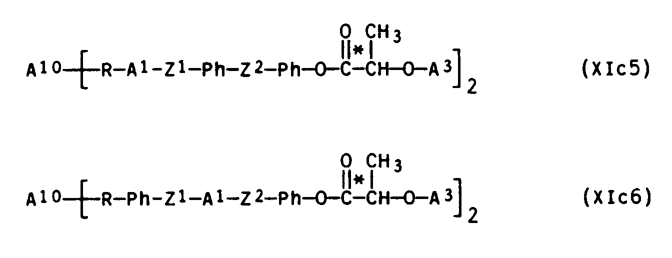

In den Formeln (XIc1)-(XIc6) bedeutet A¹⁰ einen Rest der Formel XIV:

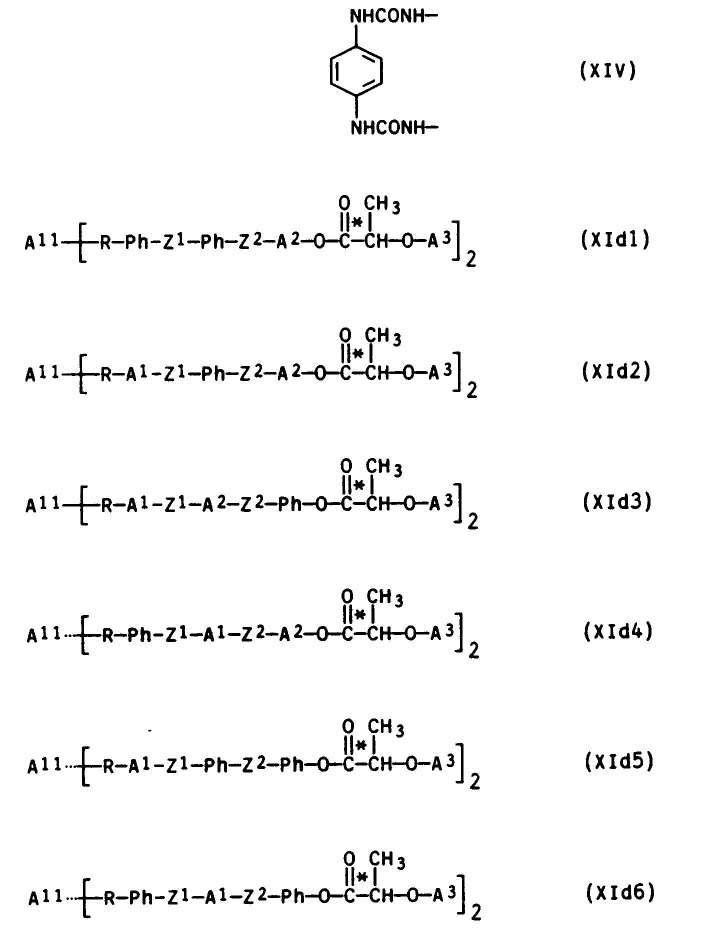

In den Formeln (XId1)-(XId6) bedeutet A¹¹ einen Rest der Formel XV:

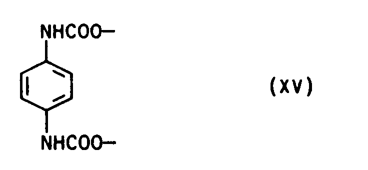

Die erfindungsgemäßen Verbindungen der Formeln I, II und XI finden als Komponenten chiraler getilteter smektischer flüssigkristalliner Phasen Verwendung.

Beispielhaft sind im folgenden konkrete Synthesen aufgeführt:

### Herstellung der monomeren Verbindungen I:

### Beispiel 1

20 g (Benzyloxi)-phenol (100 mmol) wurden in 500 ml Tetrahydrofuran nacheinander mit 16,4 g D-Phenoxipropionsäure VII (100 mmol), 1,48 g 4-Pyrrolidino-pyridin (10 mmol) und 37,1 g Dicyclohexylcarbodiimid (180 mmol) bei einer Temperatur von 5 - 10°C versetzt. Anschließend wurde das Reaktionsgemisch bis zur vollständigen Umsetzung bei Raumtemperatur weitergerührt (DC-Kontrolle). Der entstandene Niederschlag wurde abgetrennt, das Lösungsmittel unter vermindertem Druck entfernt, der Rückstand in Essigsäureethylester aufgenommen, die organische Phase mehrmals mit Wasser gewaschen, getrocknet und unter vermindertem Druck eingeengt. Das Rohprodukt wurde aus Isopropanol umkristallisiert.
Ausbeute: 30 g (≙ 87 %).

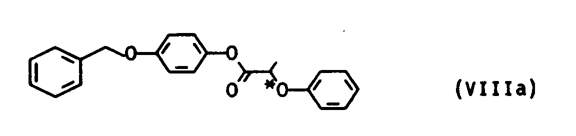

In analoger Weise wurden hergestellt:

### Beispiel 2

Ausbeute: 39 g (≙ 85 %)

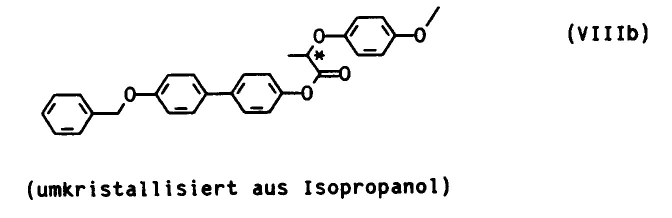

### Beispiel 3

Ausbeute: 35 g (≙ 87 %)

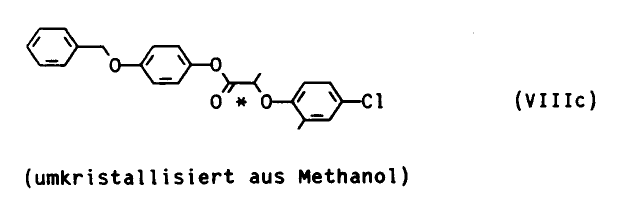

### Beispiel 4

Ausbeute: 37 g (≙ 88 %)

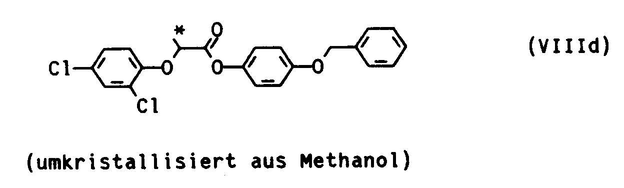

### Beispiel 5

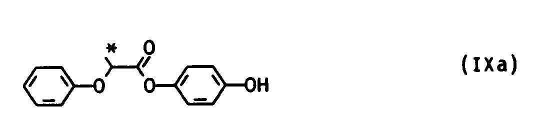

16,5 g der Verbindung VIIIa (47,4 mmol) wurden in 250 ml Ethanol mit Raney-Nickel versetzt und mit Wasserstoffgas bei Normaldruck debenzyliert.
Ausbeute: 11 g (≙ 90 %).
Fp.: 144°C

In analoger Weise wurden hergestellt:

### Beispiel 6

aus Verbindung VIIIb:
Ausbeute: 10 g (≙ 60 %)

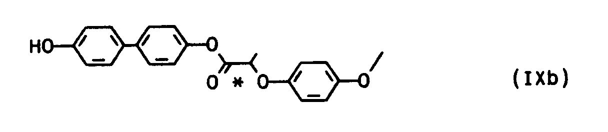

### Beispiel 7

aus Verbindung VIIIc:
Ausbeute: 10 g (≙ 70 %)

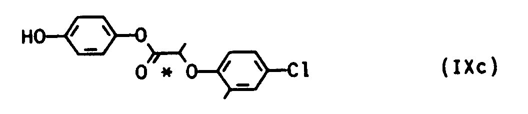

### Beispiel 8

20,2 g der Verbindung der Formel X (46,1 mmol) wurden in Tetrahydrofuran bei 0 - 5°C unter Rühren mit 12 g der Verbindung IXa (46,5 mmol), 0,62 g Pyrrolidino-pyridin (4,2 mmol) und 13,6 g Dicyclohexylcarbodiimid (66 mmol) versetzt und bei Raumtemperatur bis zur vollständigen Umsetzung (DC-Kontrolle) weiter gerührt. Der gebildete Niederschlag wurde entfernt, das Lösungsmittel unter vermindertem Druck entfernt, der Rückstand in Methylenchlorid aufgenommen, mit Wasser ausgeschüttelt, die organische Phase getrocknet, das Lösungsmittel unter vermindertem Druck entfernt, und der Rückstand an Kieselgel mit Toluol/Essigester = 10:1 v/v chromatographiert und aus Methanol umkristallisiert.
Ausbeute: 6,0 g (≙ 20 %)
¹H-NMR (CDCl₃; δ in ppm):
1,0-2,0 (m, 21 al-H), 3,8-4,2 (m, 4H, -OCH₂-), 5,0 (m, 1H, -OCOCHMeO-), 6,8-8,3 (m, 17 ar-H und 3H, -CH=CH₂)

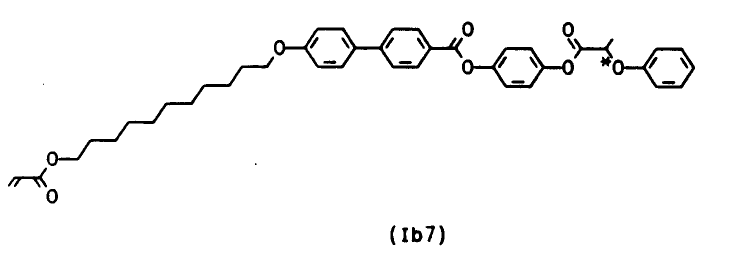

In analoger Weise wurden hergestellt:

### Beispiel 9

aus Verbindung IXa:
Ausbeute: 7,2 g (≙ 30 %)
¹H-NMR (CDCl₃; δ in ppm):
1,0-2,0 (m, 11al-H), 3,9-4,3 (m, 4H, -OCH₂-), 5,0 (m, 1H, -OCOCHMeO-), 6,7-8,2 (m, 13ar-H und 3H, -CH=CH₂)

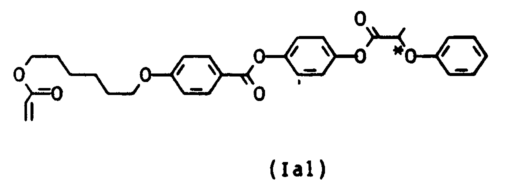

### Beispiel 10

aus Verbindung IXb:
Ausbeute: 0,2 g (≙ 3 %)
Klärtemperatur: 181°C

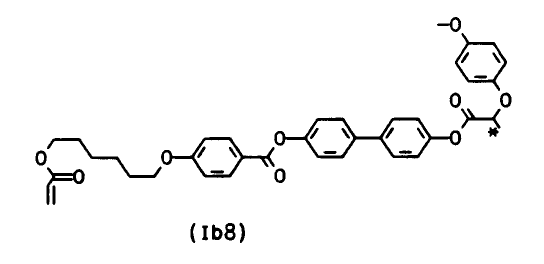

### Beispiel 11

aus Verbindung IXc:
Ausbeute: 6,1 g (≙ 21 %)
¹H-NMR (CDCl₃; δ in ppm):
1,0-2,0 (m, 18 al-H), 2,3 (s, 3H, -CH₃), 3,8-4,3 (m, 4H, -OCH₂-), 5,0 (m, 1H, -OCOCHMeO-), 5,8 (d, 1H, -CH=CH₂), 6,1 (dd, 1H, -CH=CH₂), 6,4 (d, 1H, -CH=CH₂), 6,7-8,3 (m, 15 ar-H)

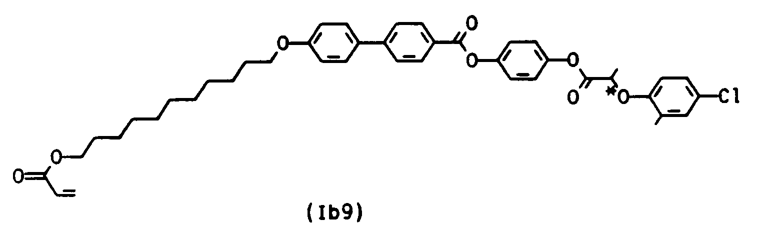

### Beispiel 12

aus Verbindung IXb:
Ausbeute: 5,7 g (≙ 17 %)
¹H-NMR (CDCl₃; δ in ppm):
1,0-2,0 (m, 21 al-H), 3,8 (s, 3H, -OCH₃-), 4,0-4,3 (m, 4H, -CH₂O-), 4,9 (q, 1H, -OCOCHMeO-), 5,8 (d, 1H, -CH=CH₂), 6,1 (dd, 1H, -CH=CH₂), 6,4 (d, 1H, -CH=CH₂), 6,8-8,3 (m, 16 ar-H)

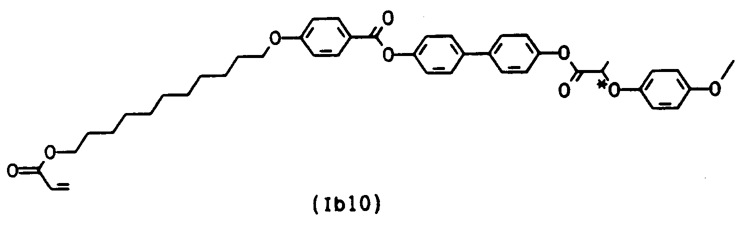

Allgemeine Verfahrensvorschrift für die Herstellung von Polyacrylaten:

Die Polymerisation erfolgte in einem Schlenkgefäß unter Schutzgasatmosphäre. Die chromatographierten und mehrfach umkristallisierten Monomere wurden in Konzentrationen von 0,1 - 0,01 mol/l in absolutem THF (Merck, > 99,5 %, Molekular-Sieb 4 Å) gelöst. Dann wurde in die Lösung während 15 Minuten Argon eingeleitet, 1 - 40 Mol.-% 2,2'-Azo-bis-(2-isobutyronitril) (aus Methanol unterhalb 40°C umkristallisiert) zugegeben, und während weiterer 15 Minuten Argon eingeleitet.

Die Lösung wurde nun auf 50°C thermostatisiert, das Schlenkgefäß verschlossen und 100 - 140 h bei dieser Temperatur gehalten. Anschließend wurde die Reaktionsmischung über Millipore Filter (Typ FG, Porengröße 0,2 µm) abgesaugt, mit Methanol (Merck, > 99,8 %) ausgefällt, abgesaugt, der Rückstand erneut in THF gelöst und mit Petroläther (Ligroin, Sdp. 30 - 75°C) gefällt. Dieser Vorgang wurde mehrere Male wiederholt. Der Rückstand wurde im Hochvakuum von Lösungsmittelresten befreit.

Nach dem obigen Verfahren wurden die folgenden Verbindungen hergestellt:

### Beispiel 13

aus Verbindung Ib7:
Ausbeute: 1,1 g
Klärtemperatur: 171°C

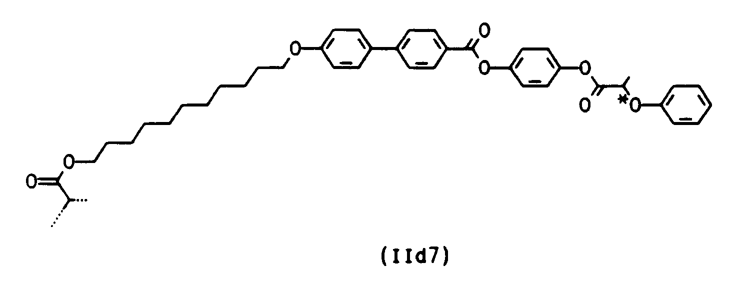

### Beispiel 14

aus Verbindung Ia1:
Ausbeute: 1,2 g
Glastemperatur: 40°C

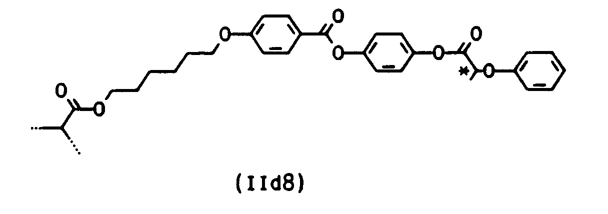

### Beispiel 15

aus Verbindung Ib9:
Ausbeute: 0,9 g
Klärtemperatur: 192°C

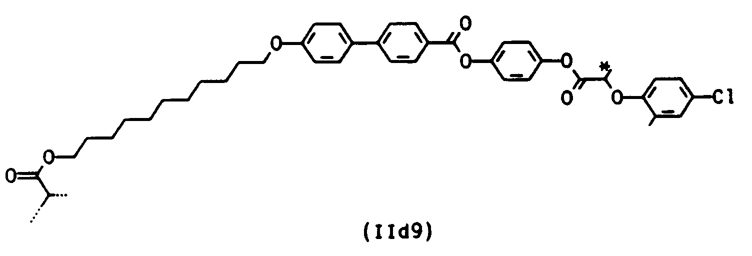

## Patentansprüche

1. Optisch aktive Verbindungen der Formel I 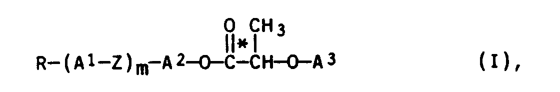 worin die Indices und die Variablen die folgende Bedeutung haben:
R eine C₁-C₁₂-Alkyl- oder -Perfluoralkyl-Gruppe, in der auch eine oder zwei nicht benachbarte CH₂- bzw. CF₂-Gruppen durch O-Atome und/oder -CO-Gruppen und/oder -CO-O-Gruppen und/oder -CH=CH-Gruppen und/oder -CH-Halogen- und/oder -CHCN-Gruppen und/oder -O-CO-CH-Halogen- und/oder -O-CO-CHCN-Gruppen ersetzt sein können, oder eine C₁-C₁₂-Alkylgruppe, die endständig eine chemisch reaktive Gruppe tragen kann und in der eine CH₂-Gruppe durch ein O-Atom ersetzt sein kann,
A¹ und A² unabhängig voneinander unsubstituiertes oder durch ein oder zwei F- und/oder Cl- und/oder Br-Atome und/oder CH₃-Gruppen und/oder CN-Gruppen substituiertes 1,4-Phenylen, worin auch eine oder zwei CH-Gruppen durch N ersetzt sein können, 1,4-Cyclohexylen, worin auch eine oder zwei nicht benachbarte CH₂-Gruppen durch O-Atome und/oder S-Atome ersetzt sein können, Piperidin-1,4-diyl, 1,4-Bicyclo(2,2,2)octylen, Naphthalin-2,6-diyl, Decahydronaphthalin-2,6-diyl oder 1,2,3,4-Tetrahydronaphthalin-2,6-diyl,
A³ gegebenenfalls substituiertes Phenyl,
Z -CO-O-, -O-CO-, -CH₂CH₂-, -OCH₂-, -CH₂O-, -C≡C- oder eine Einfachbindung und
m 0, 1, 2 oder 3,
mit der Maßgabe, daß für den Fall, daß m = 2 oder 3 ist, die Reste A¹ bzw. Z in den einzelnen Gruppen -(A¹-Z) unabhängig voneinander jeweils gleich oder verschieden sein können.

2. Optisch aktive Verbindungen nach Anspruch 1, dadurch gekennzeichnet, daß m 1 oder 2 bedeutet.

3. Optisch aktive Verbindungen nach Anspruch 2, dadurch gekennzeichnet, daß m 2 bedeutet.

4. Optisch aktive Verbindungen nach Anspruch 1, dadurch gekennzeichnet, daß Z -CO-O-, -O-CO- oder eine Einfachbindung bedeutet.

5. Optisch aktive Verbindungen nach Anspruch 1, dadurch gekennzeichnet, daß R für einen C₅-C₁₁-Alkyl oder einen C₅-C₁₁-Alkoxyrest steht.

6. Optisch aktive Verbindungen nach Anspruch 5, dadurch gekennzeichnet, daß R endständig eine chemisch reaktive Gruppe R¹ trägt.

7. Optisch aktive Verbindungen nach Anspruch 6, dadurch gekennzeichnet, daß R¹ für eine Acrylat-, Methacrylat-, Chloracrylat-, Ethenyl- oder eine Hydroxygruppe steht.

8. Optisch aktive Verbindungen nach Anspruch 1, dadurch gekennzeichnet, daß A¹ und A² für eine 1,4-Phenylengruppe stehen.

9. Optisch aktive Verbindungen nach Anspruch 1, dadurch gekennzeichnet, daß A³ für eine Phenylgruppe steht.

10. Optisch aktive Verbindungen nach Anspruch 1, dadurch gekennzeichnet, daß A³ für eine Phenylgruppe steht, die ein oder zwei voneinander unabhängige C₁-C₁₀-Alkyl-, C₁-C₁₀-Alkoxy-, Brom- oder Chlorsubstituenten trägt.

11. Polymere II, dadurch gekennzeichnet, daß sie optisch aktive Verbindungen I gemäß einem der Ansprüche 1 bis 10 einpolymerisiert enthalten.

12. Niedermolekulare, glasartig erstarrende Verbindungen, erhältlich durch chemische Reaktion von mehrfach reaktiven Verbindungen Z³ mit solchen optisch aktiven Verbindungen I, die reaktive Gruppen R¹ tragen, wobei Z³ eine mehrfach reaktive Verbindung aus der Gruppe der aliphatischen Alkohole, aliphatischen Carbonsäuren, cycloaliphatischen Alkohole mit 5 bis 6 Ringgliedern, cycloaliphatischen Carbonsäuren mit 5 bis 6 Ringgliedern, Alkoholamine, Triazinderivate oder Isocyanate bedeutet.

13. Verwendung der erfindungsgemäßen Verbindungen gemäß einem der Ansprüche 1 bis 12 allein, in Mischungen untereinander und mit anderen flüssigkristallinen und/oder nicht flüssigkristallinen Verbindungen in Form fester oder flüssigkristalliner optisch anisotroper Medien für die Darstellung und Speicherung von Information.

## Claims

1. An optically active compound of the formula I 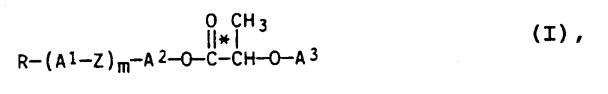 where
R is C₁-C₁₂-alkyl or -perfluoroalkyl in which one or two non-adjacent CH₂ or CF₂ groups can also be replaced by -O- and/or -CO- and/or -CO-O-and/or -CH=CH- and/or -CH-halogen- and/or -CHCN- and/or -O-CO-CH-halogen- and/or -O-CO-CHCN-, or is C₁-C₁₂-alkyl which can have a terminal chemically reactive group and in which a CH₂ group can be replaced by -O-,
A¹ and A² are each, independently of one another, 1,4-phenylene which is unsubstituted or substituted by one or two F and/or Cl and/or Br atoms and/or CH₃ groups and/or CN groups and in which one or two CH groups can also be replaced by N, 1,4-cyclohexylene in which one or two non-adjacent CH₂ groups can also be replaced by -O-and/or -S-, 1,4-piperidinediyl, 1,4-bicyclo-[2.2.2]octylene, 2,6-naphthalenediyl, decahydro-2,6-naphthalenediyl or 1,2,3,4-tetrahydro-2,6-naphthalenediyl,
A³ is unsubstituted or substituted phenyl,
Z is -CO-O-, -O-CO-, -CH₂CH₂-, -OCH₂-, -CH₂O-, -C≡C- or a single bond and
m is 0, 1, 2 or 3,
with the proviso that when m is 2 or 3 the radicals A¹ and Z in the individual -(A¹-Z) groups can each, independently of one another, be identical or different.

2. An optically active compound as claimed in claim 1, wherein m is 1 or 2.

3. An optically active compound as claimed in claim 2, wherein m is 2.

4. An optically active compound as claimed in claim 1, wherein Z is -CO-O-, -O-CO- or a single bond.

5. An optically active compound as claimed in claim 1, wherein R is C₅-C₁₁-alkyl or C₅-C₁₁-alkoxy.

6. An optically active compound as claimed in claim 5, wherein R has a terminal chemically reactive group R¹.

7. An optically active compound as claimed in claim 6, wherein R¹ is acryloyloxy, methacryloyloxy, chloroacryloyloxy, ethenyl or hydroxyl.

8. An optically active compound as claimed in claim 1, wherein A¹ and A² are each 1,4-phenylene.

9. An optically active compound as claimed in claim 1, wherein A³ is phenyl.

10. An optically active compound as claimed in claim 1, wherein A³ is phenyl which has one or two C₁-C₁₀-alkyl, C₁-C₁₀-alkoxy, bromine or chlorine substituents which are independent of one another.

11. A polymer II which contains an optically active compound I as claimed in any of claims 1 to 10 as copolymerized unit.

12. A low molecular weight compound which forms a glass-like solid, obtainable by chemical reaction of a polyreactive compound Z³, with an optically active compound I which has reactive groups R¹, where Z³ is a polyreactive compound from the group of aliphatic alcohols, aliphatic carboxylic acids, cycloaliphatic alcohols with 5 to 6 ring members, cycloaliphatic carboxylic acids with 5 to 6 ring members, alcohol amines, triazine derivatives or isocyanates.

13. The use of the compounds as claimed in any of claims 1 to 12 alone, in mixtures with one another and with other liquid-crystalline and/or non-liquid-crystal-line compounds in the form of solid or liquid-crystalline, optically anisotropic media for the presentation and storage of information.

## Revendications

1. Composés optiquement actifs de formule I 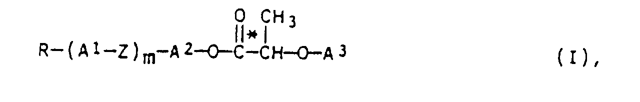 dans laquelle les indices et les variables ont la signification suivante :
R un groupement alkyle ou perfluoroalkyle en C₁-C₁₂, dans lequel un ou deux groupements CH₂, respectivement CF₂, non voisins, peuvent être remplacés par des atomes d'oxygène et/ou des groupements -CO et/ou des groupements -CO-O et/ou des groupements -CH=CH et/ou des groupements -CH-Halogène et/ou -CHCN et/ou des groupements -O-CO-CH-Halogène et/ou -O-CO-CHCN, ou un groupement alkyle en C₁-C₁₂, qui peut porter un groupement chimique réactif en bout de chaîne et dans lequel un groupement CH₂ peut être remplacé par un atome d'oxygène,
A¹ et A² indépendamment l'un de l'autre, groupements 1,4- phénylènes non substitués ou substitués par un ou deux atomes de fluor et/ou de chlore et/ou de brome et/ou des groupements CH₃ et/ou des groupements CN, dans lesquels un ou deux groupements CH peuvent être remplacés par des atomes d'azote, groupements 1,4 cyclohexylènes, dans lesquels un ou deux groupements CH₂ non voisins peuvent être remplacés par des atomes d'oxygène et/ou de soufre, groupements pipéridine -1,4- diyle, 1,4- bicyclo (2,2,2) octylène, naphtalène -2,6- diyle, décahydronaphtalène -2,6- diyle, ou 1,2,3,4- tétrahydronaphtalène -2,6-diyle,
A³ groupement phényle éventuellement substitué,
Z -CO-O-, -O-CO-, -CH₂ CH₂-, -OCH₂-, -CH₂O-, -C≡C ou une simple liaison et
m 0, 1, 2 ou 3,
sachant que, dans le cas où m vaut 2 ou 3, le groupement A¹, respectivement Z, peut être identique ou différent à chaque fois, dans les groupements individuels -(A¹-Z), indépendamment les uns des autres.

2. Composés optiquement actifs selon la revendication 1, caractérisés en ce que m vaut 1 ou 2.

3. Composés optiquement actifs selon la revendication 2, caractérisés en ce que m vaut 2.

4. Composés optiquement actifs selon la revendication 1, caractérisés en ce que Z représente -CO-O-, -O-CO- ou une simple liaison.

5. Composés optiquement actifs selon la revendication 1, caractérisés en ce que R est mis pour un groupement alkyle en C₅-C₁₁ ou alcoxy en C₅-C₁₁.

6. Composés optiquement actifs selon la revendication 5, caractérisés en ce que R porte un groupement chimique réactif R¹ en bout de chaîne.

7. Composés optiquement actifs selon la revendication 6, caractérisés en ce que R¹ est mis pour un groupement acrylate, méthacrylate, chloroacrylate, éthényle ou hydroxy.

8. Composés optiquement actifs selon la revendication 1, caractérisés en ce que A¹ et A² sont mis pour un groupement 1,4- phénylène.

9. Composés optiquement actifs selon la revendication 1, caractérisés en ce que A³ est mis pour un groupement phényle.

10. Composés optiquement actifs selon la revendication 1, caractérisés en ce que A³ est mis pour un groupement phényle, qui porte, indépendamment l'un de l'autre, un ou deux substituants alkyle en C₁-C₁₀, alcoxy en C₁-C₁₀, brome ou chlore.

11. Polymères II, caractérisés en ce qu'ils comportent des composés I optiquement actifs, selon l'une quelconque des revendications 1 à 10, en liaison polymère.

12. Composés à bas poids moléculaire et à solidification vitreuse, obtenus par réaction chimique de composés Z³ plurivalents avec des composés I optiquement actifs et qui portent des groupements réactifs R¹, où Z³ représente un composé plurivalent du groupe constitué par les alcools aliphatiques, les acides carboxyliques aliphatiques, les alcools cycloaliphatiques avec 5 à 6 maillons, les acides carboxyliques cycloaliphatiques avec 5 à 6 maillons, les aminoalcools, les dérivés de la triazine ou les isocyanates.

13. Utilisation des composés selon l'invention et selon l'une quelconque des revendications 1 à 12, seuls, en mélange entre eux ou avec d'autres composés liquides cristallins et/ou non liquides cristallins, sous forme de médium optiquement anisotrope, solide ou liquide cristallin, pour l'affichage et l'enregistrement d'informations.
